# EUROPEAN PATENT APPLICATION

(11) **EP 3 839 975 A1**
(43) Date of publication of application: **23.06.2021**
(21) Application number: 19218614.6
(22) Date of filing: 20.12.2019
(51) Int. Cl.: G16H 50/50, A61B 5/11

(54) **DEVICE MONITORING SYSTEM**

(71) Applicant: Nagravision S.A., 1033 Cheseaux-sur-Lausanne (CH)
(72) Inventor: Villegas, Karine, 1033 Cheseaux-sur -Lausanne (CH); Keomany, Jean, 1033 Cheseaux-sur -Lausanne (CH); Perrine, Jérôme, 1033 Cheseaux-sur -Lausanne (CH)
(74) Representative: Hoyng Rokh Monegier B.V.

(57) **Abstract**

Providing devices designed and/or modified based, at least in part, on a device-specific parameter and a reference model.

## Description

### FIELD OF THE INVENTION

The present disclosure generally relates to devices designed and/or modified based, at least in part, on a device-specific parameter and a reference model; to systems and methods for use in designing and/or modifying a device based, at least in part, on a device-specific parameter and a reference model.

### BACKGROUND

Products require periodic diagnostic maintenance to detect wear, predict failure and locate problems. Failure of the products results in costly productivity loss, severe and expensive damage and potentially life-threatening situations. Product failures occur because, over time, the parts that are stressed experience wear, misalignment or damage. To ensure safety or achieving desired function, products are typically replaced at conservative fixed intervals based, for example, on laboratory tests performed by the product manufacturers.

One example is in the field of joint replacement. It may be necessary to perform a joint replacement procedure on the patient as a result of, for example, disease or trauma. Once implanted, the replaced joint may undergo significant amounts of wear and/or misalignment. Once the required functionality of the replaced joint is no longer realized to its required level, a revision surgery may need to be performed on the patient. In such a revision surgery, usually, the previously implanted artificial joint is surgically removed, and a substantially the same or similar artificial joint is implanted in the patient.

Although implant devices are designed by variety of software models, which gather various data specific to patient's body parts via medical imaging devices before placed in the patient, there is no *in-vivo* analysis of the implant device in order to predict loss of functionality of the implant devices, which is specific to that particular implant device and influenced by the specific characteristics of the patient receiving the implant.

No apparent effort is being made to learn from the loss of functionality of the implant device in a specific patient and to improve the subsequent implant device to be placed on that specific patient on the basis thereto. Further, there is not enough *in-vivo* information for medical practitioners to inform a specific patient about ways of using the implant device in order to avoid or decrease loss of functionality of the implant device to an undesirable level.

Similar problems occur in various industries such as automotive or even garment industry. Products are designed based on data obtained from general parameters and laboratory tests. These products are not designed for specific users or operators based on data obtained during operation of the device and specific characteristics of the operators or users of the products. Thus, their lifespan is unpredictable and require periodic replacements or revisions. For example, certain vehicle owners exhibit aggressive or frequent braking behavior, which may cause the brake pads to wear more quickly. Brake pad life monitoring systems have been implanted that provide an alarm when the brake pad wears sufficiently. However, the same or similar brake pads are usually used as replacement. As a result, the replaced brake pads wear more likely within the same period as the previous pad due to operational behavior of its driver, which increases cost and the operations required for replacement thereof.

Thus, there is an existing need in device development field for extending life of a device and/or to reduce its frequent revision and replacement.

It is desirable to extend life of a device by designing it according to the way it is used.

It is also desirable to be able to inform a specific user how to use the device and/or how to modify its behavior in order to extend the life of the device and to reduce its replacement.

The present invention provides such devices, systems and methods that solve one or more of the problems mentioned above. Other features and advantages of the invention will be apparent from the following description and from the claims.

### BRIEF DESCRIPTION OF THE DRAWINGS

The features and advantages of the disclosure will be appreciated upon reference to the following drawing, in which:
Fig. 1 illustrates an exemplary environment in which a method in accordance with an embodiment of the present disclosure is applied on an hip implant.
Fig. 2 illustrates an exemplary environment in which a method in accordance with an embodiment of the present disclosure may be applied.
Fig. 3 shows a hardware infrastructure for implementing an embodiment.

The figure is meant for illustrative purposes only, and does not serve as restriction of the scope or the protection as laid down by the claims.

### DESCRIPTION OF ILLUSTRATIVE EMBODIMENTS

In one aspect of the present disclosure, a non-transitory computer readable storage medium is provided.

The term "non-transitory computer-readable medium" as used herein refers to any medium that comprises the actual performance of an operation (such as hardware circuits), that comprises programs and/or instructions to be provided to one or more processors for performance/implementation (such as instructions stored in a non-transitory memory), and/or that comprises instructions stored in memory. Non-transitory computer-readable media may take many forms, such as nonvolatile and volatile media, including but not limited to, a floppy disk, flexible disk, hard disk, RAM, any memory chip, any magnetic medium from which a computer instruction can be read; a CD-ROM, DVD, or any other optical medium from which a computer instruction can be read, or any other non-transitory medium from which a computer instruction can be read.

In one embodiment the programs and/or higher-level instructions in the non-transitory computer-readable medium intend to be used for one or more medical purposes. Optionally, the programs and/or higher-level instructions in the non-transitory computer-readable medium intend to be used without being part of a hardware medical device. "without being part of' means software not necessary for a hardware medical device to achieve its intended medical purpose.

In one embodiment, the non-transitory computer readable storage medium configured to store instructions that when executed cause a processor to perform comparing data representative of a device-specific parameter with a reference model. A host, a device, and/or a remote element can comprise at least one non-transitory computer-readable medium. Optionally, a device of present disclosure comprises at least one non-transitory computer-readable medium. Optionally, a remote element (e.g. a server, smartphone) comprises at least one non-transitory computer-readable medium according to present disclosure.

As used herein the term "device-specific parameter" refers to data (including various parameters) specific to a device. This data is obtained when the device is being used by a host. For example, *in vivo* data of an implant device placed in a specific patient. The device-specific parameter can be measured continuously in real time. Optionally, the device-specific parameter is measured and transmitted continuously in real time to a remote element (e.g. a server) and/or to a non-transitory computer readable storage medium. Optionally, the device comprises a data storage device and the device-specific parameters are stored in the data storage device. Optionally, the device-specific parameters are stored in a data storage device of a remote element and/or in a non-transitory computer readable storage medium.

In one embodiment, the device-specific parameter comprises at least one from the group consisting of a kinematic parameter (including linear and/or angular kinematics) of the device; spatiotemporal parameter of the device; pressure excreted on the device; torque excreted on the device; temperature of the device; and acceleratory forces (kinetics) excreted on the device.

Optionally, the kinematic parameters comprise at least one from the group consisting of movement of a component or a part of the device toward or away from the centerline of the device (e.g. abduction and adduction in case of joint implants); internal rotation of the device, part of the device or a component of the device; flexion of the device, part of the device or a component of the device; extension of the device, part of the device or a component of the device, tilt forwards, sideways, and reverse of the device, part of the device, or a component of the device.

Kinematic parameters of the device of present disclosure define the motion of points, bodies of the device, part of the device or component(s) of the device. The motion is defined relative to a three-dimensional reference frame. For example, a reference frame will be defined by defining certain point(s) within the device, for instance, a zero-movement point. A defined starting point of the kinematic reference frame allows data to be generated about the time, displacement, velocity and acceleration of the device relative to that defined reference frame. Kinematic parameters may be defined in two groups, linear kinematics and angular kinematics.

When kinematic parameters are utilized, through a linear or an angular reference frame, the data will provide information on the time, displacement, velocity and acceleration of the sensor. For example, should the kinematic reference frame be utilized in the cylinder head of a vehicle, the directional deviation, velocity and acceleration with each stroke may be measured to define the exact movement of the cylinder under certain ways of utilizing the vehicle (e.g., braking force, acceleration, speedbumps, centrifugal forces in corners).

Furthermore, the spatiotemporal parameter, optionally, includes width movement defined as the distance, in transverse plane, between a specific point on the device and a specific point on a host. For example, during walking a specific point on an artificial ankle or hip and a specific point on ankle of the other foot (measured for example in centimeters or meters). Spatiotemporal parameters, optionally, include cadence, which can be defined as the rhythm of movement of the device. For example, a rhythm of a person's walk, usually expressed in steps per minute (steps/min).Optionally, the sensors provide spatiotemporal parameters of the device. These parameters may be indicated as spatial parameters measured over a time-reference. For example, the measured parameters in an implant device may comprise cadence, width and length of a step, stride length, the percentage of single support phase, and the swing phase for a limb. In other sensors, the measured parameters may be average linear or angular velocity, number of directional deviations per time-unit.

The device-specific parameter such as kinematic and spatiotemporal parameters of the device of present disclosure can be detected, measured and analyzed by the commercially available sensors and software models. Optionally, the sensors are kinematic and spatiotemporal sensors or network of sensors such as motion sensors, e.g. accelerometer, gyroscope (measuring angular velocity), displacement sensor, linear motion sensor, angular and rotary motion sensor.

Optionally, the device-specific parameter is indicative of wear of the device, fatigue of the device, corrosion of the implant device, misalignment of the device, deformation of the device, or combinations thereof.

In one embodiment, the device-specific parameter is *in vivo* parameter of the medical device used or being used by the patient. Optionally, in one embodiment, the host is a patient and the device is an implant device; and wherein the device-specific parameter comprises a spatiotemporal parameter; wherein the spatiotemporal parameter comprises at least one from the group consisting of step width, step length, stride length, and cadence. The device-specific parameter can be generated during a time period by at least one sensor of at least one sensing unit.

As used herein, the term "device" has its art understood meaning and refers to anything that has a purpose for a host that uses the device. As used in the domain of technology, examples of devices are machines, tools, gadgets, appliances, peripherals, electronic components, mechanical components, implants, grafts, consumer products, garments, shoes and the like.

In one embodiment, the device is a medical device, optionally, an implant device. As used herein, the terms "implant" or "graft" are used interchangeably and refer to anything that is implanted in or on something else to perform a specific function. The implant can be placed inside or onto something else. For example, a skin implant is placed on or under the skin and a hip implant is placed inside the patient's body. The implant can be a piece of tissue, prosthetic device or another object implanted in or on a host. Specific examples of the implant devices include dental implants, skin implants, artificial joints such as shoulder joint, an elbow joint, a wrist joint, a hip joint, a knee joint, and an ankle joint; and any replaceable parts thereof.

The device of present disclosure can be a monoblock device or a modular device. Monoblock, One-component or single-piece implant refers to a device (e.g. implant device) that is designed in one-piece. For example, the device is designed as one piece without using screws, a linker, or locking mechanism to hold them in place. In order to replace the monoblock device, the whole device needs to be replaced. Modular or Two or more component or multiple component device refers to a device comprising a number of interchangeable and detachable components. For example, the components of a device are detachable and are hold in place by a locking mechanism.

As used herein the term "host" refers to anything with respect to whom a device is intended to be, or has been, used. The term "host" includes machines like robotics, computers, vehicles or non-machines such as humans, plants, and animals. The term "host" includes patient. The term "patient" refers to any individual (human or animal) with respect to whom a device is intended to be, or has been, used. Optionally, the patient is human, and the device is a medical device, optionally an implant device.

As used herein the term "medical device" refers an instrument, apparatus, implement, machine, contrivance, implant, in vitro reagent, or other similar or related article, including a component part, or accessory intended for use in the diagnosis of disease or other conditions, or in the cure, mitigation, treatment, or prevention of disease, in man or other animals, or intended to affect the structure or any function of the body of man or other animals.

As used herein the term "reference model" refers to predetermined and desired model, sub-model, values, standards, threshold values, or parameters available to a service provider such as device manufacturer or developer of a particular device. For example, manufacturers standard values and part replacement and repair recommendations and/or rules can also be defined in the reference model. The reference model is used here to model the (e.g. via algorithm) behavior or function of the device for the purpose of model-based device diagnoses, monitoring, modification and/or development. The reference model of present disclosure comprises at least one host-specific's characteristic. Optionally, the reference model comprises at least one host-specific characteristic and at least one predetermined value as set by the manufacturers.

In one embodiment, the reference model of present disclosure comprises imaging data collected from the patient, for example, imaging data from one or more of x-ray imaging, MRI, PET, ultrasound, and is used to qualitatively and/or quantitatively measure one or more of a patient's biological features. The measured biological features can include shape, for example, two-dimensional shape or three-dimensional shape; area, for example, surface area and/or surface contour; perimeter shape; and/or volume of, for example, the patient's cartilage, bone.

Additional host (e.g. patient)-specific measurements and information that can be used in the reference model can include, for example, joint kinematic measurements, bone density measurements, soft and connective tissues structures, skin, muscles, , and patient information, such as patient age, weight, gender, ethnicity, activity level, and overall health status.

In addition to (or optionally in place of) the above-mentioned measurements, it may be desirable to have the reference model to include measurements of the targeted joint as well as surrounding anatomical areas and/or other joints of the patient's anatomy in a weight-bearing condition. Such measurements can provide data on the alignment and/or movement of the joint and surrounding structures as well as the loading conditions of the various joint components. Such load-bearing measurements can include imaging of the patient standing, kneeling, walking and/or carrying loads of varying sizes and/or weights.

As used herein the term "host-specific parameter" refers to a variety of information which is unique to each host using or has been used the device. For example, when the host is human and the device is an implant, the host-specific information is biomechanical information, kinematic parameters, spatio-temporal parameters, family medical history information, medical conditions (e.g. allergy information, Parkinson disease, rehabilitation training effect, Frail syndrome), exercise information, drug prescriptions, skin and body conditions (e.g. skin type, skin texture, muscle tone, weight, height, etc.), age of the human or any combinations of any of the host-specific parameter.

Optionally, the reference model is stored in at least one sensing unit. Optionally, the reference model is stored in a remote element (e.g. server) or in a non-transitory computer readable storage medium according to present disclosure.

The remote element of the present disclosure is optionally located outside the host. The remote element of the present disclosure is designed to detect, read, monitor, or simply is responsive to one or more of the sensing units of the present disclosure and to produce a measurable output. For example, the remote element is a server. Optionally, at least one remote element is being designed to wirelessly power the sensing units. Optionally, at least one remote element of the present invention is designed to be any electrical filed emitting, electromagnetic wave-emitting, electromagnetic wave-detecting, and/or electromagnetic wave-activated and activating device, such as, for example, radio frequency identification (RFID) transponders or tags, microwave tags, inlays, etc. More than one remote element can be used to transmit and to receive signals to and from different sensing units placed at different locations. The output of the sensing unit and/or the remote element of the present invention may be measured and displayed by various types of consumer electronics devices, including, but not limited to, personal computers, digital video disk devices, television sets, audio reproduction systems, smart phones, and notebooks. However, in various alternate embodiments, any appropriate electronic network designed to permit communication between any desired types of electronic devices.

Optionally, in the reference model, the device characteristics (e.g. manufacturer standard values or repair and replacement recommendations) are normalized with a host-specific information.

The sensing unit of present disclosure comprises a sensor and a transmitting device configured to transmit at least data representative of the device-specific parameter. Optionally, the sensing unit comprises a data receiving device (e.g. transceiver), a processor operatively coupled to the data receiving device (e.g. transceiver) and/or a non-transitory computer readable storage medium connected to the processor. Optionally, the sensing unit is physically attached to the device or to one or more components of the device. Optionally, the sensing unit is not in physical contact with the device. Optionally, at least one sensing unit is physically attached to the device or a component of the device and at least one sensing unit is not physically attached to the device or a component of the device.

The sensing unit of present disclosure is configured to generate data representative of the device-specific parameter continuously, optionally, every second, every minute, every day, every month, and/ or months.

The non-transitory computer readable storage medium according to present disclosure configured to store instructions that when executed cause a processor to perform or implement a function.

In one embodiment, the instructions configured that when executed to cause the processor to detect and/or to monitor whether the device-specific parameter exceeds and/or is within a threshold value predetermined by the reference model based on comparison of the device-specific parameter with the reference model.

In one embodiment, the instructions configured that when executed cause a processor to perform receiving data representative of a device-specific parameter generated during a time period by a sensing unit of a device used or being used by a host.

In one embodiment, the instructions configured that when executed cause the processor to detect an approaching device failure for a future time period based at least in part on the comparison of device-specific parameter with the reference model. Device failure occurs when the required functionality of the device is no longer realized to its required threshold level or value as predetermined by the reference model.

In further embodiment, the instructions configured that when executed to cause the processor to display, on the display, the device modification or device replacement recommendation based at least in part on the comparison of device-specific parameter and the reference model.

In one embodiment, the instructions configured that when executed to cause the processor to indicate that the device is operating beyond the desired predetermined threshold level as set in a reference model (for example due to the way in which the device is being used). Accordingly, device is modified or replaced to meet the threshold level as set in the reference model. For example, the replacement of the device may be made of a tougher or more durable material at a certain point or location of the device to improve the frequency of replacement components. Optionally, the device is modified while being used in a host. For example, at least one component or part of the device becomes softer or harder or moved wirelessly.

In one embodiment, the instruction configured that when executed to cause the processor to diagnose a condition of an implant device being used by the patient, wherein the instruction configured to cause the processor to inform the patient and/or an implant device manufacturer to modify or to replace the implant device when the device-specific parameter exceeds a threshold value predetermined by the reference model.

Optionally, the instructions configured that when executed to cause a processor to perform comparing the data with the reference model continuously, optionally, every second, every minute, every day, every month, or months.

In further embodiment, the non-transitory computer readable storage medium of present disclosure is configured to store instructions that when executed cause a processor to perform comparing a data representative of an in-vivo device-specific parameter generated by a sensing unit of an implant device used or being used by a patient during a time period with a reference model, wherein the instruction configured to cause the processor to inform the patient and/or an implant device manufacturer to modify or to replace the implant device when the device-specific parameter exceeds a threshold value predetermined by the reference model, wherein the in vivo device specific parameter comprises at least one from the group consisting of a kinematic parameter of an implant device; spatiotemporal parameter of the implant device; pressure; torque; temperature; and acceleratory forces excreted by or on the implant device; wherein the reference model comprises a predetermined lifespan of the implant device normalized by a patient-specific parameter; wherein the output is indicative of whether the device-specific parameter exceeds and/or is within a threshold value predetermined by the reference model.

In another aspect of present disclosure, a server device is provided. The server device comprises a data receiving device such as a transceiver configured to receive data representative of a device-specific parameter generated during a time period by a sensing unit of a device used or being used by a host; a processor operatively coupled to the transceiver; and one or more non-transitory computer readable storage medium according to any of the paragraphs operatively connected to the processor.

In this embodiment, it is clear that any of the paragraphs of the present disclosure is incorporated by reference to the embodiments representing the server. For example, all the paragraphs of present disclosure related to a non-transitory computer readable storage medium configured to store instructions that when executed cause a processor to perform any functions mentioned above alone or in combination; sensing units; device specific parameter; host; device are incorporated by reference.

In another aspect of the present disclosure, the device according to any of the paragraphs of the present disclosure is manufactured, i.e. obtained, via the non-transitory computer readable storage media; servers, system, and methods according to present disclosure. In this embodiment, it is clear that any of the paragraphs of the present disclosure is incorporated by reference to the embodiments representing the server. For example, all the paragraphs of present disclosure related to the non-transitory computer readable storage medium configured to store instructions that when executed cause a processor to perform any functions mentioned above; servers, sensing units; device specific parameter; host; reference models; systems; methods, alone or in combination are incorporated by reference.

In one embodiment, at least a portion of the one or more structural components of the device is obtained based, at least in part, on an output generated via at least one sensing unit and/or at least one non-transitory computer readable storage medium by comparing data representative of a device-specific parameter with a reference model; wherein the device is obtained when the comparison data indicate that the device-specific parameter is within or exceeds a threshold value predetermined by the reference model. Optionally, the device is obtained when the comparison data indicate that the device-specific parameter exceeds a threshold value predetermined by the reference model.

In one embodiment, the device comprises one or more structural components and at least one sensing unit and/or at least one non-transitory computer readable storage medium, wherein at least a portion of the one or more structural components is modified (optionally wirelessly, optionally when the host is still using the device) based, at least in part, on data obtained during operation of the device and transmitted by the transmitting device, said data is based on comparison of a device-specific parameter and a reference model, wherein the device is modified when the device-specific parameter exceeds a threshold predetermined by the reference model. For example, the device does not need to be replaced but it may be adjusted or modified. For example, the device or at least one component of the device (e.g. wirelessly) becomes softer or stiffer to meet the predetermined threshold value as set by the reference model in order to extend the life of the device.

In another aspect of the present disclosure, a system is provided. All of the embodiments of present disclosure apply to this embodiment and are incorporated by reference in their entireties.

In one embodiment, the system comprises at least one sensing unit according to any of the paragraphs; at least one remote element being designed to receive data from at least one sensing unit of any of the paragraphs, and at least one non-transitory computer readable storage medium according to any of the paragraphs. Optionally, the system comprises the device according to any of the paragraphs of present disclosure. Optionally, the system comprises a device capable of displaying the output generated by at least one sensing unit and/or at least one non-transitory computer readable storage medium according to any of the paragraphs such as personal computer or smart phone.

In another aspect of present invention methods for detecting and/or monitoring a device-specific parameter such as wear of the device, fatigue of the device, corrosion of the device, misalignment of the device, deformation of the device, or combinations thereof are provided. The methods of present invention enable a processor to detect and/or to monitor whether the device-specific parameter exceeds and/or is within a threshold value predetermined by the reference model based on comparison of device-specific parameter and the reference model.

Further, the methods of present disclosure enable processor to detect an approaching device threshold value as predetermined by the reference model based at least in part on the comparison of device-specific parameter and the reference model.

The methods of present disclosure can be used for diagnosing the device-specific parameters of an implant device being used by the patient and accordingly inform the patient and/or an implant device manufacturer to modify or to replace the implant device when the device-specific parameter exceeds a threshold value predetermined by the reference model.

In one embodiment, the method of present disclosure comprises comparing data representative of a device-specific parameter generated during a time period by a sensing unit of a device used or being used by a host with a reference model; determining whether the device-specific parameter exceeds and/or is within a threshold value predetermined by the reference model based on comparison of device-specific parameter and the reference model. When the device-specific parameter exceeds, about to exceed, and/or approaching at a faster rate than recommended rate, the threshold value predetermined by the reference model, an output is generated, which accordingly informs the user (e.g. patient) or operator of the device (device is being used or has been used by the host). The user such as patient or an operator such medical practitioner or manufactures can modify the device while the device is being used or by extracting the device from the host and adjusting and modifying it. Alternatively, the device is replaced by another modified device, which takes into account the measured device-specific parameters of the original device into its modification and design.

In one embodiment, the method of present disclosure can also comprise receiving data representative of a device-specific parameter generated during a time period by a sensing unit (the sensing unit can be part of the structure of the device or outside of the structure of the device), which is used or being used by a host.

In one embodiment, the received data is coupled to a non-transitory computer readable storage medium according to any of the paragraphs in order to instruct a processor, when executed, to perform the mentioned functions.

The non-transitory computer readable storage medium; servers; systems; devices; and methods according to any of the paragraphs of present disclosure address device malfunction quickly and efficiently to reduce adverse events. They understand and capture host (e.g. patient) performance outside of the clinical and laboratory settings and enable the host (e.g. patient) engagement. Unlike manufacturers of hardware devices who modify their product every few months to years, the device of present disclosure can be modified in response to real-world performance of the host and user feedback every second to months.

For example, the non-transitory computer readable storage media; servers; systems; medical devices; and methods according to any of the paragraphs of present disclosure are able to diagnose/screen/detect a disease or condition, to aid in treatment by providing enhanced support to safe and effective use of a medical device, to aid in making a definitive diagnosis, to identify early signs of a disease or conditions, to inform the patient and medical practitioners about the available options to improve functioning and lifespan of the device, and to provide clinical information by aggregating relevant information.

The non-transitory computer readable storage media; servers; systems; medical devices; and methods according to any of the paragraphs of present disclosure can include adaptive (e.g. keeps pace with the changing environment), perfective (e.g. recoding to improve software performance), corrective (e.g. corrects discovered problems), or preventive (e.g. corrects latent faults in the software product before they become operational faults).

The term "adaptive maintenance" as used herein refers to the modification of the non-transitory computer readable storage media; servers; systems; medical devices; and/or methods according to any of the paragraphs of present disclosure, performed after delivery, to keep them usable in a changed or changing environment.

The term "perfective maintenance" as used herein refers to the modification of the non-transitory computer readable storage media; servers; systems; medical devices; and/or methods according to any of the paragraphs of present disclosure after delivery to detect and correct latent faults in them before they are manifested as failures.

The term "corrective maintenance" as used herein refers to the reactive modification of the non-transitory computer readable storage media; servers; systems; medical devices; and/or methods according to any of the paragraphs of present disclosure performed after delivery to correct discovered problems.

The term "preventive maintenance" as used herein refers to the modification of the non-transitory computer readable storage media; servers; systems; medical devices; and/or methods according to any of the paragraphs of present disclosure after delivery to detect and correct latent faults in them before they become operational faults. Examples of changes include, but are not limited to, defect fixes; aesthetic, performance or usability enhancements; and security patches.

The term "intended use / intended purpose" is the objective intent of the manufacturer regarding the use of a product, process or service as reflected in the specifications, instructions and information provided by the manufacturer.

### EXAMPLES

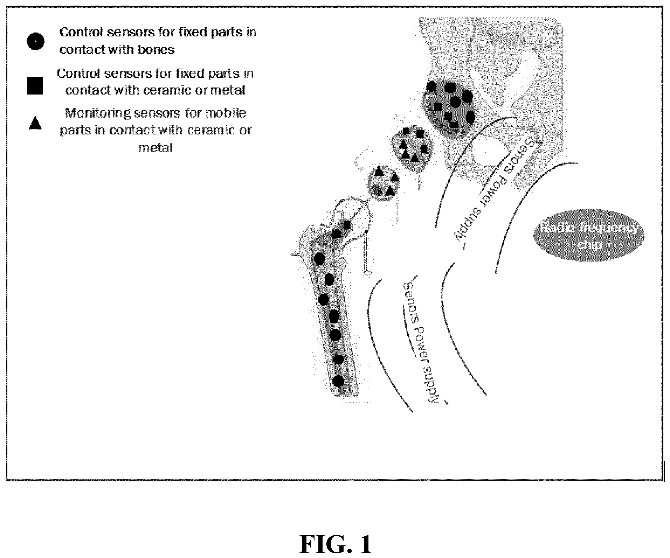

Fig. 1 illustrates an exemplary environment in which a method in accordance with an embodiment of the present disclosure may be applied.

In this example, a hip prothesis has been shown. Mobility sensors are inserted in various parts of the prothesis such as the cupule and in the tete phemorale to be able to control and/or monitor values such as mobility, pressure and wear. The sensors collect values and enable to construct geometrical model of the mechanisms/moves of each prothesis pieces and anticipate premature wear of the piece(s) by proposing solution by comparing it to a reference model, which is designed specifically for the patient wearing the prosthesis, to adapt the movements of the patient wearing the prosthesis in a way that helps to reduce its wear.

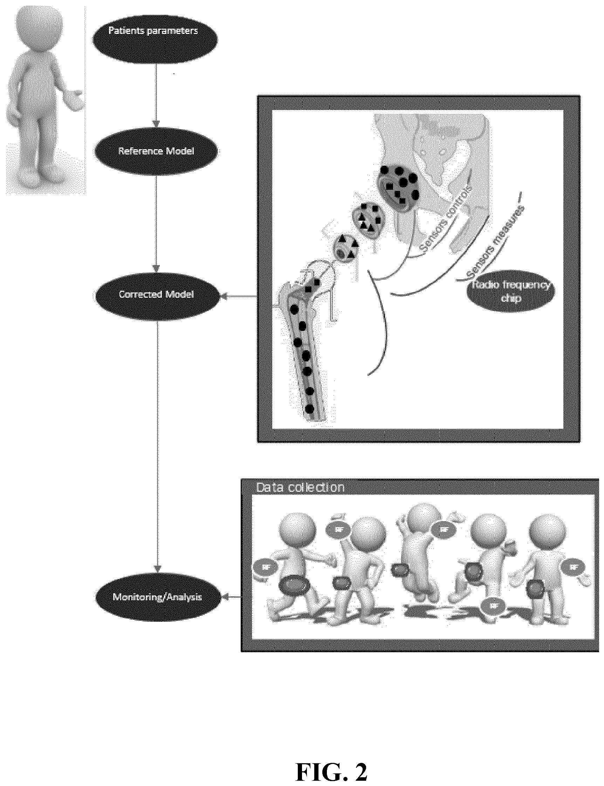

Fig. 2 illustrates another example in which a method in accordance with an embodiment of the present disclosure may be applied.

In this example, according to patient's parameters such as weight, age, height, a reference model is defined. Each piece of the prosthesis according to its function would be equipped either with sensors that control there is non mobility or with sensors that monitors locally the pressions/rotations.

Once the prosthesis is installed, the reference model is adapted according to the patient's walk. Via, for example, radio frequency, the sensors are power supplied and able to perform measures and then send them to a chip. The chip sends the data to a platform able to analyze them and compared to what was expected with each sensor according to the reference model. If the values sent by sensors do not correspond, it would be a next step for the medical staff or the prothesis manufacturer to adapt the prothesis, propose complementary solutions to the patients to try to restore the correct measures. In addition, the sensors can be integrated in the prosthesis itself and that delivers signal only when the cover layer upon them is destroyed.

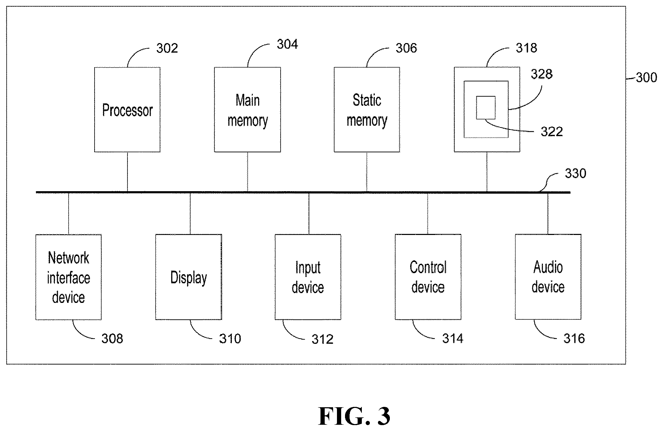

Figure 3 illustrates a block diagram of one implementation of a computing device 300 within which a set of instructions, for causing the computing device to perform any one or more of the methodologies discussed herein, may be executed. The computing device 300 may be used for elements of a system used to carry out the present disclosure.

In alternative implementations, the computing device may be connected (e.g., networked) to other machines in a Local Area Network (LAN), an intranet, an extranet, or the Internet. The computing device may operate in the capacity of a server or a client machine in a client server network environment, or as a peer machine in a peer-to-peer (or distributed) network environment.

The computing device may be a personal computer (PC), a tablet computer, a set-top box (STB), a wearable computing device, a Personal Digital Assistant (PDA), a cellular telephone, a web appliance, a server, a network router, switch or bridge, or any machine capable of executing a set of instructions (sequential or otherwise) that specify actions to be taken by that machine. Further, while only a single computing device is illustrated, the term "computing device" shall also be taken to include any collection of machines (e.g., computers) that individually or jointly execute a set (or multiple sets) of instructions to perform any one or more of the methodologies discussed herein.

The example computing device 300 includes a processing device 302, a main memory 304 (e.g., read-only memory (ROM), flash memory, dynamic random access memory (DRAM) such as synchronous DRAM (SDRAM) or Rambus DRAM (RDRAM), etc.), a static memory 306 (e.g., flash memory, static random access memory (SRAM), etc.), and a secondary memory (e.g., a data storage device 318), which communicate with each other via a bus 330.

Processing device 302 represents one or more general-purpose processors such as a microprocessor, central processing unit, or the like. More particularly, the processing device 302 may be a complex instruction set computing (CISC) microprocessor, reduced instruction set computing (RISC) microprocessor, very long instruction word (VLIW) microprocessor, processor implementing other instruction sets, or processors implementing a combination of instruction sets. Processing device 302 may also be one or more special-purpose processing devices such as an application specific integrated circuit (ASIC), a field programmable gate array (FPGA), a digital signal processor (DSP), network processor, or the like. Processing device 302 is configured to execute the processing logic (instructions 322) for performing the operations and steps discussed herein.

The computing device 300 may further include a network interface device 308. The computing device 300 also may include a video display unit 310 (e.g., a liquid crystal display (LCD) or a cathode ray tube (CRT)), an alphanumeric input device 312 (e.g., a keyboard or touchscreen), a cursor control device 314 (e.g., a mouse or touchscreen), and an audio device 316 (e.g., a speaker).

The data storage device 318 may include one or more machine-readable storage media (or more specifically one or more non-transitory computer-readable storage media 328) on which is stored one or more sets of instructions 322 embodying any one or more of the methodologies or functions described herein. The instructions 322 may also reside, completely or at least partially, within the main memory 304 and/or within the processing device 302 during execution thereof by the computing device 300, the main memory 304 and the processing device 302 also constituting computer-readable storage media.

The various methods described above may be implemented by a computer program. The computer program may include computer code arranged to instruct a computer to perform the functions of one or more of the various methods described above. The computer program and/or the code for performing such methods may be provided to an apparatus, such as a computer, on one or more computer readable media or, more generally, a computer program product. The computer readable media may be transitory or non-transitory. The one or more computer readable media could be, for example, an electronic, magnetic, optical, electromagnetic, infrared, or semiconductor system, or a propagation medium for data transmission, for example for downloading the code over the Internet. Alternatively, the one or more computer readable media could take the form of one or more physical computer readable media such as semiconductor or solid state memory, magnetic tape, a removable computer diskette, a random access memory (RAM), a read-only memory (ROM), a rigid magnetic disc, and an optical disk, such as a CD-ROM, CD-R/W or DVD

In an implementation, the modules, components and other features described herein (for example control unit 310 in relation to Figure 3) can be implemented as discrete components or integrated in the functionality of hardware components such as ASICS, FPGAs, DSPs or similar devices as part of an individualization server.

A "hardware component" is a tangible (e.g., non-transitory) physical component (e.g., a set of one or more processors) capable of performing certain operations and may be configured or arranged in a certain physical manner. A hardware component may include dedicated circuitry or logic that is permanently configured to perform certain operations. A hardware component may be or include a special-purpose processor, such as a field programmable gate array (FPGA) or an ASIC. A hardware component may also include programmable logic or circuitry that is temporarily configured by software to perform certain operations.

Accordingly, the phrase "hardware component" should be understood to encompass a tangible entity that may be physically constructed, permanently configured (e.g., hardwired), or temporarily configured (e.g., programmed) to operate in a certain manner or to perform certain operations described herein.

In addition, the modules and components can be implemented as firmware or functional circuitry within hardware devices. Further, the modules and components can be implemented in any combination of hardware devices and software components, or only in software (e.g., code stored or otherwise embodied in a machine-readable medium or in a transmission medium).

The detailed description set-forth above is provided to aid those skilled in the art in practicing the present invention. However, the invention described and claimed herein is not to be limited in scope by the specific embodiments herein disclosed because these embodiments are intended as illustration of several aspects of the invention. The embodiments set-forth above can be performed and combined with other disclosed embodiments according to the invention. Any equivalent embodiments are intended to be within the scope of this invention. Indeed, various modifications of the invention in addition to those shown and described herein will become apparent to those skilled in the art from the foregoing description which do not depart from the spirit or scope of the present inventive discovery. Such modifications are also intended to fall within the scope of the appended claims. All publications, patents, patent applications and other references cited in this application are incorporated herein by reference in their entirety for all purposes to the same extent as if each individual publication, patent, patent application or other reference was specifically and individually indicated to be incorporated by reference in its entirety for all purposes. Citation of a reference herein shall not be construed as an admission that such is prior art to the present invention.

## Claims

1. A non-transitory computer readable storage medium configured to store instructions that when executed cause a processor to perform:
comparing *in-vivo* data representative of a device-specific parameter generated during a time period by a sensing unit of an implant device used or being used by a patient with a reference model;
detecting and/or to monitoring whether the device-specific parameter exceeds and/or is within a threshold value predetermined by the reference model based on comparison of the device-specific parameter and the reference model;
wherein the predetermined threshold value as set by the reference model is generated at least based on a patient-specific parameter and a predetermined lifespan of the implant device.

2. The non-transitory computer readable storage medium according to claim 1, wherein the patient-specific parameter comprises at least one from the group consisting of patient's age, patient's weight, patient's activity level, and patient's medical condition.

3. The non-transitory computer readable storage medium according to any of the preceding claims, wherein the *in vivo* data representative of the device specific parameter comprises at least one from the group consisting of a kinematic parameter of an implant device; spatiotemporal parameter of the implant device; pressure; torque; temperature; and acceleratory forces excreted by or on the implant device.

4. The non-transitory computer readable storage medium according any of the preceding claims, wherein the instructions configured that when executed to cause a processor to perform receiving data representative of a device-specific parameter generated during a time period by a sensing unit of a device used or being used by the patient.

5. The non-transitory computer readable storage medium according to any of the preceding claims, wherein the instruction configured that when executed to cause the processor to display, on the display, the device modification or device replacement recommendation based at least in part on the comparison of device-specific parameter and the reference model.

6. The non-transitory computer readable storage medium according to any of the preceding claims, wherein the sensing unit is configured to generate data representative of the device-specific parameter every second, every minute, every day, every month, and/ or months; and wherein the instructions configured that when executed to cause a processor to perform comparing the data with the reference model every second, every minute, every day, every month, or months.

7. The non-transitory computer readable storage medium according to any of the preceding claims, wherein the reference model comprises a predetermined lifespan of the device normalized by the patient-specific parameter.

8. The non-transitory computer readable storage medium according to any of the preceding claims, wherein the instruction configured to cause the processor to diagnose a condition of the implant device being used by the patient, wherein the instruction configured to cause the processor to inform the patient and/or an implant device manufacturer to modify or to replace the implant device when the device-specific parameter exceeds a threshold value predetermined by the reference model.

9. The non-transitory computer readable storage medium according to any of the preceding claims, wherein the device-specific parameter comprises a spatiotemporal parameter; wherein the spatiotemporal parameter comprises at least one from the group consisting of step width, step length, stride length, and cadence of the patient.

10. A device comprising:
a transceiver configured to receive *in vivo* data representative of a device-specific parameter generated during a time period by a sensing unit of an implant device used or being used by a patient;
a processor operatively coupled to the transceiver;
one or more non-transitory computer readable storage medium according to claims 1-9 operatively connected to the processor.

11. A method for detecting and/or monitoring a condition of an implant device comprising:
comparing *in-vivo* data representative of a device-specific parameter generated during a time period by a sensing unit of an implant device used or being used by a patient with a reference model;
detecting and/or to monitoring whether the device-specific parameter exceeds and/or is within a threshold value predetermined by the reference model based on comparison of the device-specific parameter and the reference model;
wherein the predetermined threshold value as set by the reference model is generated at least based on a patient-specific parameter and a predetermined lifespan of the implant device.

12. The method of claim 11, wherein the condition of the implant device includes at least one from the group consisting of wear of the device, fatigue of the device, corrosion of the device, deformation of the device.

13. The method of claim 11, wherein the patient-specific parameter comprises at least one from the group consisting of patient's age, patient's weight, patient's activity level, and patient's medical condition.

14. The method of claim 11, wherein the *in vivo* data representative of the device specific parameter comprises at least one from the group consisting of a kinematic parameter of an implant device; spatiotemporal parameter of the implant device; pressure; torque; temperature; and acceleratory forces excreted by or on the implant device.

15. The method of claim 11, further comprising: displaying, on the display, the device modification or device replacement recommendation based at least in part on the comparison of device-specific parameter and the reference model.
